# EUROPEAN PATENT SPECIFICATION

(11) **EP 3 228 329 B1**
(45) Date of publication and mention of the grant of the patent: **08.06.2022**
(21) Application number: 16397510.5
(22) Date of filing: 06.04.2016
(51) Int. Cl.: A61L 15/28, A61L 15/44, A61L 15/60, A61P 17/02

(54) **A METHOD FOR PREPARING A MEDICAL PRODUCT COMPRISING NANOFIBRILLAR CELLULOSE AND A MEDICAL PRODUCT**
VERFAHREN ZUR HERSTELLUNG EINES MEDIZINISCHEN PRODUKTS MIT NANOFIBRILLÄRER CELLULOSE UND VERFAHREN ZUR HERSTELLUNG EINES MEDIZINISCHEN PRODUKTS
PROCÉDÉ DE PRÉPARATION DE PRODUIT MÉDICAL COMPRENANT DE LA CELLULOSE NANOFIBRILLAIRE ET UN TEL PRODUIT

(43) Date of publication of application: 11.10.2017
(73) Proprietor: UPM-Kymmene Corporation, 00100 Helsinki (FI)
(72) Inventor: LUUKKO, Kari, 02660 Espoo (FI); KOSONEN, Mika, 53550 Lappeenranta (FI)
(74) Representative: Berggren Oy

(56) References cited:
- EP-A1- 3 187 195
- WO-A1-2011/116069
- WO-A1-2013/176633
- WO-A1-2014/128354
- WO-A1-2015/101711

## Description

The present application relates to a method for preparing a medical product comprising nanofibrillar cellulose with an impregnating process. The application also relates to a medical product comprising nanofibrillar cellulose.

### Background

Nanofibrillar cellulose refers to isolated cellulose fibrils or fibril bundles derived from cellulose raw material. Nanofibrillar cellulose is based on a natural polymer that is abundant in nature. Nanofibrillar cellulose has a capability of forming viscous gel in water (hydrogel).

Nanofibrillar cellulose production techniques are based on grinding (or homogenization) of aqueous dispersion of pulp fibers. The concentration of nanofibrillar cellulose in dispersions is typically very low, usually around 0.3-5%. After the grinding or homogenization process, the obtained nanofibrillar cellulose material is a dilute viscoelastic hydrogel.

There is also interest in making structural products from nanofibrillar cellulose by removing water. WO 2015/101711 describes a medical product comprising nanofibrillar cellulose and a method, wherein nanofibrillar cellulose is deposited onto a filter substrate layer having micro-scale recesses and/or protrusions, followed by dewatering procedures, wherein the liquid is drained from the nanofibrillar cellulose dispersion by the effect of lowered pressure through the patterned substrate layer. The dewatered nanofibrillar polysaccharide membrane can be removed from the filter layer or kept on it to form a multi-layered product.

### Summary

The present disclosure provides a method for preparing a medical product, said method comprising
- providing an aqueous dispersion of nanofibrillar cellulose,
- providing a layer of gauze,
- impregnating the layer of gauze by soaking throughout with the aqueous dispersion of nanofibrillar cellulose so that the nanofibrillar cellulose is distributed evenly in the gauze and no separate layers are formed from the nanofibrillar cellulose on any surface of the gauze, and
- dewatering the impregnated gauze, to obtain the medical product.

The present disclosure provides a dewatered medical product comprising a layer of gauze impregnated thoroughly with nanofibrillar cellulose so that the nanofibrillar cellulose is distributed evenly in the gauze and no separate layers are formed from the nanofibrillar cellulose on any surface of the gauze.

The present disclosure provides a medical dressing or a patch, comprising said medical product.

The present disclosure provides the medical product for use for covering and/or treating skin wounds or other damages.

The present disclosure provides a cosmetic product, such as a dressing or a patch, comprising said medical product.

The main embodiments are characterized in the independent claims. Various embodiments are disclosed in the dependent claims. The embodiments recited in dependent claims and in the description are mutually freely combinable unless otherwise explicitly stated.

The impregnated medical product of the embodiments provides enhanced mechanical strength and other properties, such as high tear strength (tear resistance), especially at moist conditions. By combining a supporting and reinforcing structure, such as a dressing fabric, i.e. the gauze, with nanofibrillar cellulose an impregnated product is formed. The fabric creates a continuous supporting network and the strength of the network is not significantly affected by moist conditions.

Certain advantageous properties of the impregnated medical products include flexibility, elasticity and remouldability. If the nanofibrillar cellulose contains moisture, it may also show suitable permeability. These properties are useful for example when the impregnated product is used as a dressing for healing wounds, or in other medical applications, such as for delivering therapeutic or cosmetic agents.

Flexibility is a feature which is desired in many applications, such as in medical applications. Flexible patches and dressings comprising nanofibrillar cellulose are useful for applying onto skin, for example for covering wounds and other damages or injuries, such as burns.

The relatively low amount and the even distribution of the nanofibrillar cellulose in the product have effects to flexifibility, elasticity, remouldability and rigidity. The rigidity of the impregnated product is relatively low and the product has an open structure which provides suitable air and/or liquid permeability.

The flexibility or elasticity (elongation) of the impregnated product can also be affected with the choice of the gauze. The nanofibrillar cellulose itself has a limited flexibility and elasticity, especially when dry. For this reason it is important to match the gauze and the network of nanofibrillar cellulose to obtain a balance between the elastic properties of gauze and nanofibrillar cellulose network.

The impregnated medical products of the embodiments also provide high absorption capacity and absorption speed, which properties are desired in medical applications such as wound healing and the like. Large sheets may be prepared which may be used for covering large areas.

The impregnated medical products described herein are useful in medical applications, wherein the materials comprising nanofibrillar cellulose are in contact with living tissue. It was discovered that nanofibrillar cellulose provides advantageous properties when it is applied for example onto skin. The products containing nanofibrillar cellulose as described herein are highly biocompatible with the living tissue and provide several advantageous effects. Without binding to any specific theory, it is believed that the impregnated medical product comprising nanofibrillar cellulose provides a very hydrophilic surface, which, when applied against a skin or other tissue, for example a skin graft wound, absorbs and retains water from the tissue and forms a water film between the medical product and the tissue promoting the healing of the wound. The medical product may be also be moistened to enhance the effect.

When the impregnated products are used for covering wounds or other damages or injuries, for example in products such as plasters, dressings, medical patches or parts of plasters, patches or dressings, several effects are provided. The usability of the products is good as the product may be applied and removed easily without being damaged, for example torn. The product may also be cut into a desired size and shape without affecting the properties thereof. When used for covering wounds the material of the impregnated product acts as an artificial skin, which protects the wound and will come loose when the wound heals. The impregnated product will not attach to a damaged skin in such irreversible way as conventional materials, which are usually very difficult to remove without damaging the healed area. The conditions between the impregnated product and the skin facilitate the healing of a damaged area.

The impregnated medical products of the embodiments are especially advantageous in the treatment of grafts, such as skin graft. The impregnated product may be used for covering the graft area and it acts as a protective layer. As the graft heals, the impregnated product forms a scab-like structure, which promotes the healing.

The impregnated products may be used for controllably and effectively delivering agents, such as therapeutic or cosmetic agents, to a patient or user.

### Brief description of the drawings

The embodiments will be explained in the following with reference to the appended drawings, where
- Figure 1: shows an example of pond size press for impregnating a gauze
- Figure 2: shows an example of size press for impregnating a gauze
- Figure 3: shows an example of an arrangement having an impregnation reservoir and a pair of nip rollers

### Detailed description

In this specification, percentage values, unless specifically indicated otherwise, are based on weight (w/w). If any numerical ranges are provided, the ranges include also the upper and lower values.

The embodiments provide a dispersion comprising nanofibrillar cellulose and optionally other ingredients, such as sodium chloride, non-nanofibrillar pulp or therapeutic or cosmetic agents or other agents. One example provides a dispersion comprising nanofibrillar cellulose and non-nanofibrillar pulp. The amount of the non-nanofibrillar pulp in the dispersion may be in the range of 0.1-60% (w/w), for example 0.1-50% (w/w), of total cellulose. The dispersion may be used for manufacturing the impregnated medical products described herein.

The terms "medical product", "impregnated product" or "impregnated medical product", or more particularly "medical product impregnated with nanofibrillar cellulose", which terms may be used interchangeably, refer to a product comprising a layer of gauze treated with nanofibrillar cellulose as described in the embodiments. The medical product may also be called as a medical structure. The impregnated product may be obtained with the preparation methods described in the embodiments.

The medical products obtained with the impregnation process differ from products obtained by layering methods, for example products obtained by laminating. Such a layered product contains separate layers which may be detected from the final products for example by dying and/or using microscopic methods, and the separate layers may be even separated by peeling. In a product obtained by impregnating process described herein the nanofibrillar cellulose is evenly distributed as a coating on the fibers of the gauze. The nanofibrillar cellulose coating is practically on separate fibers of the gauze, and it is very thin, so the dried nanofibrillar cellulose does not form a separate layer on the gauze but it is completely integrated to it, i.e. the product is non-layered. This affects to the properties of the final product. For example the rigidity of the impregnated product is lower than a layered product. Further the product obtained by impregnating has more open structure having a high air and liquid permeability. When using impregnating process substantially low amounts of nanofibrillar cellulose may be used. The nanofibrillar portion of the product is practically inseparable from the gauze.

The term "medical" refers to a product or use wherein the product is used or is suitable for medical purposes. A medical product may be sterilized, or it is sterilisable, for example by using temperature, pressure, moisture, chemicals, radiation or a combination thereof. The product may be for example autoclaved, or other methods using high temperature may be used, in which cases the product should tolerate high temperatures over 100°C, for example at least 121°C or 134°C. In one example the product is autoclaved at 121°C for 15 minutes. Also UV sterilization may be used. A medical product may also be suitable for example for cosmetic purposes.

### Starting materials for preparing a dispersion comprising nanofibrillar cellulose

One starting material comprises nanofibrillar cellulose, which comprises or consists of cellulose fibrils having diameter in the submicron range. It forms a self-assembled hydrogel network even at low concentrations. These gels of nanofibrillar cellulose are highly shear thinning and pseudoplastic in nature.

One optional further starting material comprises non-nanofibrillar pulp. Such pulp is in general conventional or regular pulp or cellulose and it may be also called as macrofibrillar pulp or macrofibrillar cellulose. In one example the non-nanofibrillar pulp is unrefined or moderately refined pulp, which may be characterized for example by pulp freeness.

Said two main starting materials may also be called as fractions, such as a nanofibrillar cellulose fraction and a non-nanofibrillar pulp fraction. The nanofibrillar cellulose fraction is usually the main fraction of the cellulosic material of the dispersion for preparing the impregnated product, for example comprising 80-99.9% (w/w) of the dry weight of total cellulose. However, in one example the dispersion does not contain any non-nanofibrillar pulp, i.e. the amount of non-nanofibrillar pulp is 0%. The non-nanofibrillar pulp is usually the minor fraction or portion of the cellulosic material of the dispersion. In one example the nanofibrillar cellulose dispersion comprises an amount of non-nanofibrillar pulp in the range of 0.1-60% (w/w) of total cellulose, for example in the range of 0.1-50% (w/w), 0.1-40% (w/w), 0.1-30% (w/w), 0.1-20% (w/w), 0.1-10% (w/w), 0.5-10% (w/w), 1-10% (w/w), 0.5-5% (w/w), 1-5% (w/w), 0.5-3% (w/w) or 1-3% (w/w) of total cellulose. "Total cellulose" as used herein refers to the dry weight of the total cellulose in the dispersion.

The final dispersion comprising nanofibrillar cellulose may contain additional ingredients, usually in minor amounts. In one example the dry matter of the dispersion contains less than 1% (w/w) of additional ingredients, for example less than 0.5%, or less than 0.2%, or less than 0.1% of the total dry matter.

In one example the dispersion comprises non-modified nanofibrillar cellulose, and optionally comprises an amount of non-nanofibrillar chemical pulp in the range of 0.1-10% (w/w) of total cellulose, or in another range disclosed above.

### Nanofibrillar cellulose

The nanofibrillar cellulose is prepared normally from cellulose raw material of plant origin. The raw material may be based on any plant material that contains cellulose. The raw material may also be derived from certain bacterial fermentation processes. In one example the plant material is wood. Wood may be from softwood tree such as spruce, pine, fir, larch, douglas-fir or hemlock, or from hardwood tree such as birch, aspen, poplar, alder, eucalyptus, oak, beech or acacia, or from a mixture of softwoods and hardwoods. In one example the nanofibrillar cellulose is obtained from wood pulp. In one example the nanofibrillar cellulose is obtained from hardwood pulp. In one example the hardwood is birch. In one example the nanofibrillar cellulose is obtained from softwood pulp.

The nanofibrillar cellulose is preferably made of plant material. In one example the fibrils are obtained from non-parenchymal plant material. In such case the fibrils may be obtained from secondary cell walls. One abundant source of such cellulose fibrils is wood fibres. The nanofibrillated cellulose is manufactured by homogenizing wood-derived fibrous raw material, which may be chemical pulp. Cellulose fibers are disintegrated to produce fibrils which have the diameter of only some nanometers, which is 50 nm at the most and gives a dispersion of fibrils in water. The fibrils may be reduced to size where the diameter of most of the fibrils is in the range of only 2-20 nm. The fibrils originating from secondary cell walls are essentially crystalline with degree of crystallinity of at least 55%. Such fibrils may have different properties than fibrils originated from primary cell walls, for example the dewatering of fibrils originating from secondary cell walls may be more challenging.

As used herein, the term "nanofibrillar cellulose" refers to cellulose fibrils or fibril bundles separated from cellulose-based fiber raw material. These fibrils are characterized by a high aspect ratio (length/diameter): their length may exceed 1 µm, whereas the diameter typically remains smaller than 200 nm. The smallest fibrils are in the scale of so-called elementary fibrils, the diameter being typically in the range of 2-12 nm. The dimensions and size distribution of the fibrils depend on the refining method and efficiency. Nanofibrillar cellulose may be characterized as a cellulose-based material, in which the median length of particles (fibrils or fibril bundles) is not greater than 50 µm, for example in the range of 1-50 µm, and the particle diameter is smaller than 1 µm, suitably in the range of 2-500 nm. In case of native nanofibrillar cellulose, in one example the average diameter of a fibril is in the range of 5-100 nm, for example in the range of 10-50 nm. Nanofibrillar cellulose is characterized by a large specific surface area and a strong ability to form hydrogen bonds. In water dispersion, the nanofibrillar cellulose typically appears as either light or turbid gel-like material. Depending on the fiber raw material, nanofibrillar cellulose may also contain small amounts of other wood components, such as hemicellulose or lignin. The amount is dependent on the plant source. Often used parallel names for nanofibrillar cellulose include nanofibrillated cellulose (NFC) and nanocellulose.

Different grades of nanofibrillar cellulose may be categorized based on three main properties: (i) size distribution, length and diameter (ii) chemical composition, and (iii) rheological properties. To fully describe a grade, the properties may be used in parallel. Examples of different grades include native (or non-modified) NFC, oxidized NFC (high viscosity), oxidized NFC (low viscosity), carboxymethylated NFC and cationized NFC. Within these main grades, also sub-grades exist, for example: extremely well fibrillated vs. moderately fibrillated, high degree of substitution vs. low, low viscosity vs. high viscosity etc. The fibrillation technique and the chemical pre-modification have an influence on the fibril size distribution. Typically, non-ionic grades have wider fibril diameter (for example in the range of 10-100 nm, or 10-50 nm) while the chemically modified grades are a lot thinner (for example in the range of 2-20 nm). Distribution is also narrower for the modified grades. Certain modifications, especially TEMPO-oxidation, yield shorter fibrils.

Depending on the raw material source, e.g. hardwood (HW) vs. softwood (SW) pulp, different polysaccharide composition exists in the final nanofibrillar cellulose product. Commonly, the non-ionic grades are prepared from bleached birch pulp, which yields high xylene content (25% by weight). Modified grades are prepared either from HW or SW pulps. In those modified grades, the hemicelluloses are also modified together with the cellulose domain. Most probably, the modification is not homogeneous, i.e. some parts are more modified than others. Thus, detailed chemical analysis is not possible - the modified products are always complicated mixtures of different polysaccharide structures.

In an aqueous environment, a dispersion of cellulose nanofibers forms a viscoelastic hydrogel network. The gel is formed at relatively low concentrations of, for example, 0.05-0.2% (w/w) by dispersed and hydrated entangled fibrils. The viscoelasticity of the NFC hydrogel may be characterized, for example, with dynamic oscillatory rheological measurements.

Regarding rheology, the nanofibrillar cellulose hydrogels are shear-thinning materials, which means that their viscosity depends on the speed (or force) by which the material is deformed. When measuring the viscosity in a rotational rheometer, the shear-thinning behavior is seen as a decrease in viscosity with increasing shear rate. The hydrogels show plastic behavior, which means that a certain shear stress (force) is required before the material starts to flow readily. This critical shear stress is often called the yield stress. The yield stress can be determined from a steady state flow curve measured with a stress controlled rheometer. When the viscosity is plotted as function of applied shear stress, a dramatic decrease in viscosity is seen after exceeding the critical shear stress. The zero shear viscosity and the yield stress are the most important rheological parameters to describe the suspending power of the materials. These two parameters separate the different grades quite clearly and thus enable classification of the grades.

The dimensions of the fibrils or fibril bundles are dependent on the raw material and the disintegration method. Mechanical disintegration of the cellulose raw material may be carried out with any suitable equipment such as a refiner, grinder, disperser, homogenizer, colloider, friction grinder, pin mill, rotor-rotor dispergator, ultrasound sonicator, fluidizer such as microfluidizer, macrofluidizer or fluidizer-type homogenizer. The disintegration treatment is performed at conditions wherein water is sufficiently present to prevent the formation of bonds between the fibers.

In one example the disintegration is carried out by using a disperser having at least one rotor, blade or similar moving mechanical member, such as a rotor-rotor dispergator, which has at least two rotors. In a disperser the fiber material in dispersion is repeatedly impacted by blades or ribs of rotors striking it from opposite directions when the blades rotate at the rotating speed and at the peripheral speed determined by the radius (distance to the rotation axis) in opposite directions. Because the fiber material is transferred outwards in the radial direction, it crashes onto the wide surfaces of the blades, i.e. ribs, coming one after the other at a high peripheral speed from opposite directions; in other words, it receives several successive impacts from opposite directions. Also, at the edges of the wide surfaces of the blades, i.e. ribs, which edges form a blade gap with the opposite edge of the next rotor blade, shear forces occur, which contribute to the disintegration of the fibers and detachment of fibrils. The impact frequency is determined by the rotation speed of the rotors, the number of the rotors, the number of blades in each rotor, and the flow rate of the dispersion through the device.

In a rotor-rotor dispergator the fiber material is introduced through counter-rotating rotors, outwards in the radial direction with respect to the axis of rotation of the rotors in such a way that the material is repeatedly subjected to shear and impact forces by the effect of the different counter-rotating rotors, whereby it is simultaneously fibrillated. One example of a rotor-rotor dispergator is an Atrex device.

Another example of a device suitable for disintegrating is a pin mill, such as a multi-peripheral pin mill. One example of such device, as described in US 6202946 B1, includes a housing and in it a first rotor equipped with collision surfaces; a second rotor concentric with the first rotor and equipped with collision surfaces, the second rotor being arranged to rotate in a direction opposite to the first rotor; or a stator concentric with the first rotor and equipped with collision surfaces. The device includes a feed orifice in the housing and opening to the center of the rotors or the rotor and stator, and a discharge orifice on the housing wall and opening to the periphery of the outermost rotor or stator.

In one example the disintegrating is carried out by using a homogenizer. In a homogenizer the fiber material is subjected to homogenization by an effect of pressure. The homogenization of the fiber material dispersion to nanofibrillar cellulose is caused by forced through-flow of the dispersion, which disintegrates the material to fibrils. The fiber material dispersion is passed at a given pressure through a narrow through-flow gap where an increase in the linear velocity of the dispersion causes shearing and impact forces on the dispersion, resulting in the removal of fibrils from the fiber material. The fiber fragments are disintegrated into fibrils in the fibrillating step.

As used herein, the term "fibrillation" generally refers to disintegrating fiber material mechanically by work applied to the particles, where cellulose fibrils are detached from the fibers or fiber fragments. The work may be based on various effects, like grinding, crushing or shearing, or a combination of these, or another corresponding action that reduces the particle size. The energy taken by the refining work is normally expressed in terms of energy per processed raw material quantity, in units of e.g. kWh/kg, MWh/ton, or units proportional to these. The expressions "disintegration" or "disintegration treatment" may be used interchangeably with "fibrillation".

The fiber material dispersion that is subjected to fibrillation is a mixture of fiber material and water, also herein called "pulp". The fiber material dispersion may refer generally to whole fibers, parts (fragments) separated from them, fibril bundles, or fibrils mixed with water, and typically the aqueous fiber material dispersion is a mixture of such elements, in which the ratios between the components are dependent on the degree of processing or on the treatment stage, for example number of runs or "passes" through the treatment of the same batch of fiber material.

One way to characterize the nanofibrillar cellulose is to use the viscosity of an aqueous solution containing said nanofibrillar cellulose. The viscosity may be, for example, Brookfield viscosity or zero shear viscosity.

In one example the apparent viscosity of the nanofibrillar cellulose is measured with a Brookfield viscometer (Brookfield viscosity) or another corresponding apparatus. Suitably a vane spindle (number 73) is used. There are several commercial Brookfield viscometers available for measuring apparent viscosity, which all are based on the same principle. Suitably RVDV spring (Brookfield RVDV-III) is used in the apparatus. A sample of the nanofibrillar cellulose is diluted to a concentration of 0.8% by weight in water and mixed for 10 min. The diluted sample mass is added to a 250 ml beaker and the temperature is adjusted to 20°C±1°C, heated if necessary and mixed. A low rotational speed 10 rpm is used.

The nanofibrillar cellulose provided as a starting material in the method may be characterized by the viscosity it provides in a water solution. The viscosity describes, for example, the fibrillation degree of the nanofibrillar cellulose. In one embodiment the nanofibrillar cellulose when dispersed in water provides a Brookfield viscosity of at least 2000 mPa·s, such as at least 3000 mPa·s, measured at a consistency of 0.8% (w/w) and at 10 rpm. In one embodiment the nanofibrillar cellulose, when dispersed in water, provides a Brookfield viscosity of at least 10000 mPa·s measured at a consistency of 0.8% (w/w) and at 10 rpm. In one example the nanofibrillar cellulose, when dispersed in water, provides a Brookfield viscosity of at least 15000 mPa·s measured at a consistency of 0.8% (w/w) and at 10 rpm. Examples of Brookfield viscosity ranges of said nanofibrillar cellulose when dispersed in water include 2000-20000 mPa·s, 3000-20000 mPa·s, 10000-20000 mPa·s, 15000-20000 mPa·s, 2000-25000 mPa·s, 3000-25000 mPa·s, 10000-25000 mPa·s, 15000-25000 mPa·s, 2000-30000 mPa·s, 3000-30000 mPa s, 10000-30000 mPa·s, and 15000-30000 mPa·s, measured at a consistency of 0.8% (w/w) and at 10 rpm.

In one embodiment the nanofibrillar cellulose comprises non-modified nanofibrillar cellulose. In one embodiment the nanofibrillar cellulose is non-modified nanofibrillar cellulose. It was found out that the drainage of non-modified nanofibrillar cellulose was significantly faster than with for example anionic grade. Non-modified nanofibrillar cellulose generally has a Brookfield viscosity in the range of 2000-10000 mPa·s, measured at a consistency of 0.8% (w/w) and at 10 rpm.

The disintegrated fibrous cellulosic raw material may be modified fibrous raw material. Modified fibrous raw material means raw material where the fibers are affected by the treatment so that cellulose nanofibrils are more easily detachable from the fibers. The modification is usually performed to fibrous cellulosic raw material which exists as a suspension in a liquid, that is, pulp.

The modification treatment to the fibers may be chemical or physical. In chemical modification the chemical structure of cellulose molecule is changed by chemical reaction ("derivatization" of cellulose), preferably so that the length of the cellulose molecule is not affected but functional groups are added to β-D-glucopyranose units of the polymer. The chemical modification of cellulose takes place at a certain conversion degree, which is dependent on the dosage of reactants and the reaction conditions, and as a rule it is not complete so that the cellulose will stay in solid form as fibrils and does not dissolve in water. In physical modification anionic, cationic, or non-ionic substances or any combination of these are physically adsorbed on cellulose surface. The modification treatment may also be enzymatic.

The cellulose in the fibers may be especially ionically charged after the modification, because the ionic charge of the cellulose weakens the internal bonds of the fibers and will later facilitate the disintegration to nanofibrillar cellulose. The ionic charge may be achieved by chemical or physical modification of the cellulose. The fibers may have higher anionic or cationic charge after the modification compared with the starting raw material. Most commonly used chemical modification methods for making an anionic charge are oxidation, where hydroxyl groups are oxidized to aldehydes and carboxyl groups, sulphonization and carboxymethylation. A cationic charge in turn may be created chemically by cationization by attaching a cationic group to the cellulose, such as quaternary ammonium group.

In one embodiment the nanofibrillar cellulose comprises chemically modified nanofibrillar cellulose, such as anionically modified nanofibrillar cellulose or cationically modified nanofibrillar cellulose. In one embodiment the nanofibrillar cellulose is anionically modified nanofibrillar cellulose. In one example the nanofibrillar cellulose is cationically modified nanofibrillar cellulose. In one embodiment the anionically modified nanofibrillar cellulose is oxidized nanofibrillar cellulose. In one example the anionically modified nanofibrillar cellulose is sulphonized nanofibrillar cellulose. In one example the anionically modified nanofibrillar cellulose is carboxymethylated nanofibrillar cellulose.

The cellulose may be oxidized. In the oxidation of cellulose, the primary hydroxyl groups of cellulose may be oxidized catalytically by a heterocyclic nitroxyl compound, for example 2,2,6,6-tetramethylpiperidinyl-1-oxy free radical, generally called "TEMPO". The primary hydroxyl groups (C6-hydroxyl groups) of the cellulosic β-D-glucopyranose units are selectively oxidized to carboxylic groups. Some aldehyde groups are also formed from the primary hydroxyl groups. When the fibers of oxidized cellulose so obtained are disintegrated in water, they give stable transparent dispersion of individualized cellulose fibrils, which may be, for example, of 3-5 nm in width. With oxidized pulp as the starting medium, it is possible to obtain nanofibrillar cellulose where Brookfield viscosity measured at a consistency of 0.8% (w/w) is at least 10000 mPa·s, for example in the range of 10000-30000 mPa·s.

Whenever the catalyst "TEMPO" is mentioned in this disclosure, it is evident that all measures and operations where "TEMPO" is involved apply equally and analogously to any derivative of TEMPO or any heterocyclic nitroxyl radical capable of catalyzing selectively the oxidation of the hydroxyl groups of C6 carbon in cellulose.

In one embodiment such chemically modified nanofibrillar cellulose, when dispersed in water, provides a Brookfield viscosity of at least 10000 mPa·s measured at a consistency of 0.8% (w/w) and at 10 rpm. In one example such chemically modified nanofibrillar cellulose, when dispersed in water, provides a Brookfield viscosity of at least 15000 mPa·s measured at a consistency of 0.8% (w/w) and at 10 rpm. In one example such chemically modified nanofibrillar cellulose, when dispersed in water, provides a Brookfield viscosity of at least 18000 mPa·s measured at a consistency of 0.8% (w/w) and at 10 rpm. Examples of anionic nanofibrillar celluloses used have a Brookfield viscosity in the range of 13000-15000 mPa·s or 18000-20000 mPa·s, or even up to 25000 mPa·s, depending on the degree of fibrillation.

In one example the nanofibrillar cellulose is TEMPO oxidized nanofibrillar cellulose. It provides high viscosity at low concentrations, for example a Brookfield viscosity of at least 20000 mPa·s, even at least 25000 mPa·s, measured at a consistency of 0.8% (w/w) and at 10 rpm. In one example the Brookfield viscosity of TEMPO oxidized nanofibrillar cellulose is in the range of 20000-30000 mPa·s, such as 25000-30000 mPa·s, measured at a consistency of 0.8% (w/w) and at 10 rpm.

In one example the nanofibrillar cellulose comprises chemically unmodified nanofibrillar cellulose. In one example such chemically unmodified nanofibrillar cellulose, when dispersed in water, provides a Brookfield viscosity of at least 2000 mPa·s, or at least 3000 mPa·s, measured at a consistency of 0.8% (w/w) and at 10 rpm.

The nanofibrillar cellulose may also be characterized by the average diameter (or width), or by the average diameter together with the viscosity, such as Brookfield viscosity or zero shear viscosity. In one embodiment said nanofibrillar cellulose has a number average diameter of a fibril in the range of 1-100 nm. In one example said nanofibrillar cellulose has a number average diameter of a fibril in the range of 1-50 nm. In one example said nanofibrillar cellulose has a number average diameter of a fibril in the range of 2-15 nm, such as TEMPO oxidized nanofibrillar cellulose.

The diameter of a fibril may be determined with several techniques, such as by microscopy. Fibril thickness and width distribution may be measured by image analysis of the images from a field emission scanning electron microscope (FE-SEM), a transmission electron microscope (TEM), such as a cryogenic transmission electron microscope (cryo-TEM), or an atomic force microscope (AFM). In general AFM and TEM suit best for nanofibrillar cellulose grades with narrow fibril diameter distribution.

In one example a rheometer viscosity of the nanofibrillar cellulose dispersion is measured at 22°C with a stress controlled rotational rheometer (AR-G2, TA Instruments, UK) equipped with a narrow gap vane geometry (diameter 28 mm, length 42 mm) in a cylindrical sample cup having a diameter of 30 mm. After loading the samples to the rheometer they are allowed to rest for 5 min before the measurement is started. The steady state viscosity is measured with a gradually increasing shear stress (proportional to applied torque) and the shear rate (proportional to angular velocity) is measured. The reported viscosity (=shear stress/shear rate) at a certain shear stress is recorded after reaching a constant shear rate or after a maximum time of 2 min. The measurement is stopped when a shear rate of 1000 s⁻¹ is exceeded. This method may be used for determining the zero-shear viscosity.

In one example the nanofibrillar cellulose, when dispersed in water, provides a zero shear viscosity ("plateau" of constant viscosity at small shearing stresses) in the range of 1000-100000 Pa·s, such as in the range of 5000-50000 Pa·s, and a yield stress (shear stress where the shear thinning begins) in the range of 1-50 Pa, such as in the range of 3-15 Pa, determined by rotational rheometer at a consistency of 0.5% (w/w) by weight in aqueous medium.

Turbidity is the cloudiness or haziness of a fluid caused by individual particles (total suspended or dissolved solids) that are generally invisible to the naked eye. There are several practical ways of measuring turbidity, the most direct being some measure of attenuation (that is, reduction in strength) of light as it passes through a sample column of water. The alternatively used Jackson Candle method (units: Jackson Turbidity Unit or JTU) is essentially the inverse measure of the length of a column of water needed to completely obscure a candle flame viewed through it.

Turbidity may be measured quantitatively using optical turbidity measuring instruments. There are several commercial turbidometers available for measuring turbidity quantitatively. In the present case the method based on nephelometry is used. The units of turbidity from a calibrated nephelometer are called Nephelometric Turbidity Units (NTU). The measuring apparatus (turbidometer) is calibrated and controlled with standard calibration samples, followed by measuring of the turbidity of the diluted NFC sample.

In one turbidity measurement method, a nanofibrillar cellulose sample is diluted in water, to a concentration below the gel point of said nanofibrillar cellulose, and turbidity of the diluted sample is measured. Said concentration where the turbidity of the nanofibrillar cellulose samples is measured is 0.1%. HACH P2100 Turbidometer with a 50 ml measuring vessel is used for turbidity measurements. The dry matter of the nanofibrillar cellulose sample is determined and 0.5 g of the sample, calculated as dry matter, is loaded in the measuring vessel, which is filled with tap water to 500 g and vigorously mixed by shaking for about 30 s. Without delay the aqueous mixture is divided into 5 measuring vessels, which are inserted in the turbidometer. Three measurements on each vessel are carried out. The mean value and standard deviation are calculated from the obtained results, and the final result is given as NTU units.

One way to characterize nanofibrillar cellulose is to define both the viscosity and the turbidity. Low turbidity refers to small size of the fibrils, such as small diameter, as small fibrils scatter light poorly. In general as the fibrillation degree increases, the viscosity increases and at the same time the turbidity decreases. This happens, however, until a certain point. When the fibrillation is further continued, the fibrils finally begin to break and cannot form a strong network any more. Therefore, after this point, both the turbidity and the viscosity begin to decrease.

In one example the turbidity of anionic nanofibrillar cellulose is lower than 90 NTU, for example from 3 to 90 NTU, such as from 5 to 60, for example 8-40 measured at a consistency of 0.1% (w/w) in aqueous medium, and measured by nephelometry. In one example the turbidity of native nanofibrillar may be even over 200 NTU, for example from 10 to 220 NTU, such as from 20 to 200, for example 50-200 measured at a consistency of 0.1% (w/w) in aqueous medium, and measured by nephelometry. To characterize the nanofibrillar cellulose these ranges may be combined with the viscosity ranges of the nanofibrillar cellulose, such as nanofibrillar cellulose which, when dispersed in water, provides a Brookfield viscosity of at least 2000 mPa·s, such as at least 10000 mPa·s, for example at least 15000 mPa·s measured at a consistency of 0.8% (w/w) and at 10 rpm.

The starting material for the preparation process is usually nanofibrillar cellulose obtained directly from the disintegration of some of the above-mentioned fibrous raw material and existing at a relatively low concentration homogeneously distributed in water due to the disintegration conditions. The starting material may be an aqueous gel at a concentration of 0.2-5%, such as 0.3-2.0%, for example in the range of 0.3-0.5%. With TEMPO oxidized gel the concentration may be lower, such as 0.2-2.0%. The gel of this type contains thus a great amount of water which may be removed

A relative small amount of non-nanofibrillar cellulose in a dispersion comprising mainly nanofibrillar cellulose speeds up the draining time of the dispersion, for example in the manufacture of membranes or the impregnated products. For example a share of one percent of non-nanofibrillar cellulose of the total cellulose could speed up the drainage even by about 50%, but at the minimum by about 15-20%. As the drying of nanofibrillar cellulose is in general time-consuming and laborious, the drying process can be facilitated without substantially affecting to the properties of the product derived from the nanofibrillar cellulose.

This enables drying of nanofibrillar cellulose of relatively low consistency to a dry matter level. It is therefore possible to prepare nanofibrillar cellulose products in a time that is feasible in view of industrial production.

The non-nanofibrillar pulp refers to pulp which is not disintegrated into nanofibrillar form, or which contains mainly non-nanofibrillar cellulose. In general the non-nanofibrillar pulp is wood pulp.

In one example the non-nanofibrillar pulp is unrefined or moderately refined pulp, which may be characterized for example by the pulp freeness, which measures the drainability of a pulp suspension. In general the freeness decreases with refining.

One example of defining the pulp properties comprises defining the drainability of a pulp suspension in water in terms of the Schopper-Riegler (SR) number (ISO 5267-1). The Schopper-Riegler test is designed to provide a measure of the rate at which a dilute suspension of pulp may be dewatered. It has been shown that the drainability is related to the surface conditions and swelling of the fibres, and constitutes a useful index of the amount of mechanical treatment to which the pulp has been subjected. The Schopper-Riegler number scale is a scale on which a discharge of 1 000 ml corresponds to a SR number of zero and zero discharge to a SR number of 100. In one example the non-nanofibrillar pulp has a SR number in the range of 11-52.

Another method for the determination of drainability in terms of the Canadian Standard Freeness (CSF) number is specified in ISO 5267-2. CSF has been developed as a measure of groundwood quality. Generally, CSF decreases with refining, and it is sensitive to fines and water quality. Usually there is a correlation with the freeness and the length of the fibers: the lower the freeness, also the lower the fiber length. In one example the non-nanofibrillar pulp has a CSF number in the range of 200-800 ml.

The non-nanofibrillar pulp may be mechanical or chemical pulp. In one example the non-nanofibrillar pulp is chemical pulp. Even though mechanical pulp may be used, chemical pulp is more pure material and may be used in a wide variety of applications. Chemical pulp lack the pitch and resin acids present in mechanical pulp, and it is more sterile or easily sterilisable. Further, chemical pulp is more flexible and provides advantageous properties for example in medical patches or dressings and other materials applied on living tissue.

In one example the non-nanofibrillar pulp is softwood pulp. Examples of softwood include spruce, pine or cedar. Softwood pulp contains longer fibers than hardwood pulp, such as over 2 mm long, which provide advantageous reinforcing properties in the final products, such as enhanced tear strength.

In one example the non-nanofibrillar pulp is chemical softwood pulp. In chemical softwood pulp the fiber length has been maintained thereby obtaining a mechanically durable but flexible material.

In one example the dispersion is a non-modified nanofibrillar cellulose dispersion comprising non-nanofibrillar chemical pulp. In one example the dispersion is a non-modified nanofibrillar cellulose dispersion comprising non-nanofibrillar chemical pulp in the range of 0.1-10% (w/w) of total cellulose.

In one example the dispersion comprises non-modified nanofibrillar cellulose and a portion of non-nanofibrillar chemical softwood pulp. In one example the dispersion comprises non-modified nanofibrillar cellulose obtained from hardwood and a portion of non-nanofibrillar chemical softwood pulp.

### Preparation of the dispersion comprising nanofibrillar cellulose

The method for preparing the dispersion comprising nanofibrillar cellulose comprises first providing nanofibrillar cellulose and optionally any auxiliary agents, such as non-nanofibrillar pulp or other agents, and then forming a dispersion containing thereof in desired amounts. In one example the dispersion is an aqueous dispersion.

Strong water retention is typical for nanofibrillar cellulose since water is bound to the fibrils through numerous hydrogen bonds. Consequently, reaching a desired dry matter content of a product comprising nanofibrillar cellulose may require a long drying time. Conventional methods such as vacuum filtration may involve several hours. Low consistency of the nanofibrillar cellulose dispersion favors the formation of thin coatings with small variations in grammage over the surface of the coating. On the other hand this will increase the amount of water that has to be removed during drying.

The problem in mechanical water removal at slow rate is assumed to be the ability of nanofibrillar cellulose hydrogel to form a very dense and impermeable nanoscale membrane around itself, for example during dewatering such as filtration. The formed shell prevents diffusion of water from the gel structure, which leads to very slow concentration rates. The same applies to evaporation where the skin formation blocks the evaporation of water.

Due to the properties of the nanofibrillar cellulose hydrogels, either of native (chemically non-modified) or chemically modified cellulose, formation of membranes or other products of uniform structure in short times that are suitable to industrial production is very challenging. In the present examples the water removal from a nanofibrillar cellulose hydrogel was improved.

The addition of a small amount of non-nanofibrillar pulp enhances the drainage of the liquid from the dispersion of nanofibrillar cellulose, which would otherwise be very challenging and time-consuming. However, to further enhance the drainage reduced pressure (vacuum) and heat may be used in combination. Further pressure may be used in combination with reduced pressure and/or heat.

Cellulose obtained through N-oxyl mediated catalytic oxidation (e.g. through 2,2,6,6-tetramethyl-1-piperidine N-oxide) or carboxymethylated cellulose are specific examples of anionically charged nanofibrillar cellulose where the anionic charge is due to a dissociated carboxylic acid moiety. These anionically charged nanofibrillar cellulose grades are potential starting materials for the preparation of impregnated product, because high quality nanofibrillar cellulose dispersions are easy to manufacture from the chemically modified pulp. The anionically charged nanofibrillar cellulose grades may be pretreated by lowering the pH of the dispersion by adding acid. This pretreatment reduces the water retention capacity. For example by lowering the pH of the nanofibrillar cellulose dispersion to below 3 the drying time using the above-described methods can be reduced. In one example the nanofibrillar cellulose dispersion where the cellulose contains anionically charged groups is pretreated by lowering its pH, whereafter the pretreated nanofibrillar cellulose dispersion is supplied at the lowered pH on the filter fabric.

The starting concentration of the nanofibrillar cellulose dispersion, usually aqueous dispersion, which is used for treating the gauze, such as in an impregnation step, may be in the range of 0.1-10%. However, it is usually not higher than 5%, for example in the range of 0.3-5.0%, for example in the range of 0.8-1.2%. This is usually the initial concentration of the nanofibrillar cellulose at the exit of the manufacturing process where it is manufactured by disintegrating fibrous raw material. However, it is possible that the nanofibrillar cellulose dispersion is diluted with a liquid from the initial concentration (concentration of the product from the manufacturing process) to a suitable starting concentration to ensure that it is distributed or impregnated evenly into the gauze. Depending on the characteristic viscosity of the nanofibrillar cellulose grade, the starting concentration may be lower or higher, and it may be in the range of 0.1-10%. Higher concentrations may be used for low-viscosity grades, which may be spread uniformly on the filter fabric despite the high concentration. The nanofibrillar cellulose issues as aqueous nanofibrillar cellulose from a manufacturing process where the fibrous starting material suspended in water is disintegrated. Draining of the liquid out of the nanofibrillar cellulose dispersion may be called "dewatering" in the case of water or aqueous solution.

Auxiliary agents for enhancing the manufacturing process or improving or adjusting the properties of the product may be included in the nanofibrillar cellulose dispersion. Such auxiliary agents may be soluble in the liquid phase of the dispersion, they may form an emulsion or they may be solid. Auxiliary agents may be added already during the manufacturing of the nanofibrillar cellulose dispersion to the raw material or added to a nanofibrillar cellulose dispersion before the impregnation. The auxiliary agents may be also added to the final product, for example by impregnating. Examples of auxiliary agents include therapeutic and cosmetic agents and other agents affecting to the properties of the product or to the properties of the active agents, such as surfactants, plasticizers, emulsifiers or the like. In one example the dispersion contains one or more salts, which may be added to enhance the properties of the final product or to facilitate water removal from the product in the manufacturing process. One example of the salt is sodium chloride. The salt may be included in an amount in the range of 0.01-1.0% (w/w) of the dry matter in the dispersion. The final product may also be dipped or soaked in a solution of sodium chloride, such as in an aqueous solution of about 0.9% sodium chloride. Desired sodium chloride content in the final product may be in the range of 0.5-1%, such as about 0.9%, of the volume of the wet product.

Compared with dewatering of nanofibrillar cellulose dispersions where the cellulose is native cellulose, dewatering of nanofibrillar cellulose dispersions where the cellulose is anionically charged cellulose is even more time-consuming because water is bound very strongly to the cellulose. Nanofibrillar cellulose containing anionically charged groups can be for example chemically modified cellulose that contains carboxyl groups as a result of the modification. Cellulose obtained through N-oxyl mediated catalytic oxidation (e.g. through 2,2,6,6-tetramethyl-1-piperidine N-oxide, known by abbreviation "TEMPO") or carboxymethylated cellulose are examples of anionically charged nanofibrillar cellulose where the anionic charge is due to a dissociated carboxylic acid moiety. When making products from nanofibrillar cellulose containing anionic groups, the total drying time is expected be many times the total drying time with nanofibrillar cellulose where the cellulose is unmodified, mainly due to the higher water retention capacity and higher viscosity of the anionically charged nanofibrillar cellulose.

The dewatering properties of these anionically charged nanofibrillar cellulose grades may be considerably improved by pretreating the nanofibrillar cellulose dispersion by an acid. When the nanofibrillar cellulose contains anionically charged groups that act as bases (acid moieties in dissociated from), as is the case with oxidized cellulose and carboxymethylated cellulose, lowering the pH with acid will convert these groups to undissociated form, the electrostatic repulsion between the fibrils is no more effective, and the water-fibril-interaction is changed in a way that favors the dewatering of the dispersion (water retention capacity of the dispersion is reduced). The pH of the anionically charged nanofibrillar cellulose dispersion is lowered below 4, preferably below 3, to improve the dewatering properties.

In an example anionically charged nanofibrillar cellulose dispersion which was obtained from "TEMPO" oxidized pulp needed a dewatering time under vacuum of roughly 100 minutes at original (unadjusted) pH, when the target grammage of the membrane was 20 grams per square meter. When the pH of the dispersion was lowered to 2 with HCI before the dewatering, the dewatering time in the same conditions was about 30 seconds, that is, the time was reduced to 0.5% of the original. The dispersion becomes visibly aggregated (fibril flocks are formed) when the pH is lowered, which is believed to be one reason for faster dewatering because water flows more easily between the aggregates.

### Preparation of the impregnated medical product

Disclosed is a method for preparing a medical product, said method comprising providing an aqueous dispersion of nanofibrillar cellulose, which may be a dispersion described herein, and providing a layer of gauze, which may be a gauze described herein.

The method comprises treating, for example impregnating, the layer of gauze with the aqueous dispersion of nanofibrillar cellulose. This may be carried out by providing the dispersion in a basin or the like and immersing or dipping the gauze into the dispersion. The gauze is kept in the dispersion for a time period suitable for letting the dispersion to impregnate the gauze, and then the gauze is removed from the dispersion. Impregnation as used herein refers to a process wherein a gauze is filled or soaked throughout, or substantially throughout, with a dispersion comprising nanofibrillar cellulose. In the impregnation the gauze will be filled, imbued, permeated or saturated with the dispersion, either partially or completely.

The "impregnating" as used herein refers to a non-layering or non-laminating treatment or process, wherein a dispersion of nanofibrillar cellulose is contacted with the layer of gauze to impregnate the gauze at least partially. In the impregnation the gauze is contacted with the impregnation solution practically from both sides at the same time, which enables the dispersion to penetrate the gauze from both sides of the gauze. According to the claimed invention, the gauze is impregnated thoroughly, so that the nanofibrillar cellulose is distributed evenly in the gauze and no separate layers are formed on any surface of the gauze, i.e. no detectable or visible layers are formed on a surface of the gauze. Techniques for forming a layered structure, such as layering or coating methods, for example blade coating, and also spraying techniques, are excluded from the method of the present invention.

Next the wet gauze may be pressed to remove excess dispersion and liquid, and to facilitate the penetration of the dispersion into the structure of the gauze.

This facilitates the even distribution of the nanofibrillar cellulose in the gauze. The properties of the gauze may however have an effect to the penetration of the nanofibrillar cellulose into the gauze, for example in the case wherein the structure or the material of the gauze is different on different sides. In one embodiment the method therefore comprises pressing the impregnated gauze, which may be carried out with any suitable pressing method and/or device, to obtain the medical product.

In one embodiment the pressing is carried out in a nip, or more particularly in a nip roll. A nip refers to the contact area where two opposing rolls meet, such as in a press or calender. Nip rolls or pinch rolls may be powered rolls and they are usually used to press two or more sheets together to form a laminated product. In one example one roll is powered and the other one is freely movable. In the present embodiments however they are used to press the impregnated gauze so the obtained product is not a laminated product. The high pressure created at the nip point brings impregnated gauze into intimate contact, and can squeeze out any bubbles or blisters. Nip roller units can also be used as pullers for material being pulled off of rolls or being fed between operations. Nip rolls are sometimes called squeeze rolls, pinch rolls or even wringers. Nip rolls may be used in several arrangements, such as pond size press and size press. The nip rolls may be overlapping so that the freely movable roll on top forms the pressure against the gauze fed into the nip point. The nip rolls may be for example steel rolls, which may have fine grooving. Using nip rolls was found very effective for facilitating the penetration of the dispersion into the gauze and simultaneously removing excess dispersion from the gauze. Nip rolls are very useful in an industrial scale process, wherein a long gauze sheet is fed immediately from impregnation to the nip rolls and further to a next step, such as to a dewatering step.

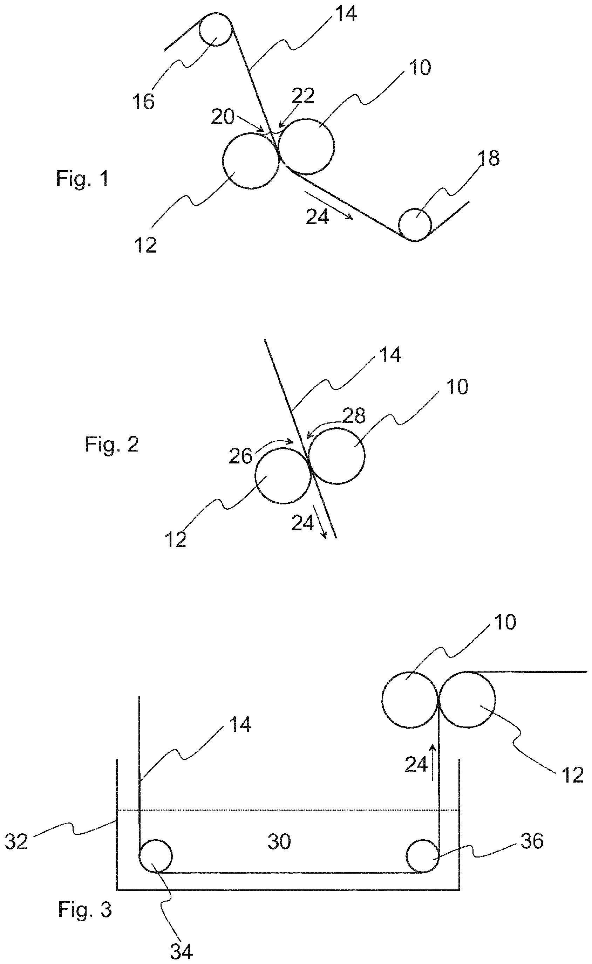

In one example the pressing is carried out in a pond press, more particularly a pond size press. This has been illustrated in Figure 1, wherein a gauze 14 runs via a first roller 16 to a pair of nip rollers 10, 12 and further to second roller 18 to a direction indicated by an arrow 24. Impregnation solution is provided immediately before the nip rollers 10, 12 to the locations formed by the nip rollers 10, 12 indicated by arrows 20, 22. The gauze is impregnated and immediately pressed in the nip.

In one example the pressing is carried out in a size press. This has been illustrated in Figure 2, wherein a gauze 14 runs through a pair of nip rollers 10, 12 to a direction indicated by an arrow 24. Impregnation solution is provided from the surface of the nip rollers 10, 12 as indicated by the arrows 26, 28, to impregnated the gauze 14.

In one example the gauze 14 is first soaked in an impregnation solution or dispersion 30 in a reservoir 32, and the fed into a pair of nip rolls 10, 12 to a direction indicated by an arrow 24, as illustrated in Figure 3. When the nip rolls 10, 12 are horizontally adjacent, the pressed gel will flow back to the impregnation reservoir. There are two rollers 34, 36 in the reservoir to guide the gauze 14 to the impregnation solution or dispersion 30. The impregnated gauze may be next further lead into a dewatering step. It is also possible to feed to dewatered gauze back to the impregnation reservoir 32. The impregnation reservoir may be the same as before or it may be different, in which case the impregnating solution or dispersion may have the same or different composition. For example an auxiliary agent, such as a pharmaceutical or cosmetic agent, may be included in the first impregnation solution or dispersion, or impregnation run, and there is no such an agent in the subsequent impregnation runs, or the concentration of the agent is different in the subsequent runs, for example the concentration is lower or higher in a subsequent run. It is also possible to include a different auxiliary agent in a subsequent impregnation run. Each impregnation run may include one or more different auxiliary agent. In one example the auxiliary agent is present only in the last impregnation run. With this method it is possible to prepare products having sustained or controlled release properties.

The treated gauze is finally dewatered. In one embodiment the method comprises dewatering the pressed impregnated gauze. This is carried out after the pressing step, or if there are several impregnating and pressing steps, after the last pressing step.

The dewatering may be carried out by non-contact drying, such as with an infrared dryer, floating dryer or impingement dryer, or by contact drying, such as with a press dryer, cylinder dryer or belt dryer. Air impingement drying involves blowing hot air (such as at 300°C) in gas burners at high velocity against the wet sheet. In belt drying, the product is dried in a drying chamber by contact with a continuous hot steel band which is heated either by steam or hot gas. The water from the band is evaporated due to the heat from the band.

When drying cylinder is used the surface of the product will be smooth and the drying is cost efficient. In one example the product is dewatered in a press dryer wherein the product is placed between a Teflon plate and a cloth or fabric, and also heat is applied.

The impregnation and the dewatering may be done once, or the steps may be repeated, if necessary to maximize saturation and/or even distribution of the dispersion in the gauze. The steps of impregnating and dewatering, optionally with a pressing step in between, together may be called for example as an impregnation run. A specific property, such as a grammage of the product, may be desired. In such case the impregnation run is repeated until the medical product has reached the desired grammage. Therefore in one example the impregnating and dewatering are repeated at least once, i.e. the impregnating and dewatering are carried out at least twice. In one example the impregnating and dewatering are carried out several times, such as 2-6 times, for example 2, 3, 4, 5 or 6 times, or more. In one embodiment the impregnating and dewatering are repeated until the medical product has reached a grammage in the range of 25-80 g/m², such as 30-70 g/m², for example 35-65 g/m², or any other grammage disclosed herein.

The gauze as used herein refers to any suitable gauze, such as a fabric, a cloth or the like material comprising fibers. The gauze may be woven or nonwoven, sterile or nonsterile, plain or impregnated, or fenestrated (perforated or with slits), or a combination thereof. The gauze may be provided as a gauze sheet or fabric or the like.

In one example the gauze is woven. By one definition a woven gauze is a thin, translucent fabric with a loose open weave. In technical terms a woven gauze is a weave structure in which the weft yarns are arranged in pairs and are crossed before and after each warp yarn keeping the weft firmly in place. The gauze may comprise natural fibers, semi-synthetic fibers or synthetic fibers, such as viscose, rayon, polypropylene, polyester and the like, or combinations thereof, for example a viscose-polyester mixture or a mixture of cellulose (pulp) and polypropylene and/or polyester. When used as a medical dressing, gauze may be made of cotton. The gauze may also act as a pad of a patch. In one embodiment the gauze is viscose-polyester gauze, for example non-woven. Such a non-woven gauze is very porous and permeable and it is moderately elastic providing irreversible elongation in one direction.

In one embodiment the gauze is nonwoven. Nonwoven gauze comprises fibers pressed together to resemble a weave, which provides improved wicking and greater absorbent capacity. Compared to woven gauze, this type of gauze produces less lint and has the benefit of leaving fewer fibers behind in a wound when removed. Examples of nonwoven gauze dressings include gauzes made of polyester, viscose, or blends of these fibers which are stronger, bulkier, and softer than woven pads.

The gauze used in the embodiments may comprise absorbing material, for example to enable the medical product to absorb exudate, to soak up blood, plasma, and other fluids exuded from the wound and containing them in one place. The gauze may also stem bleeding and to help sealing a wound. The gauze may also absorb a therapeutic agent or other agent.

In one example the gauze comprises natural fibers or natural-fiber-based material, such as cotton, cellulose, linen, silk or the like. Natural fibers provide free hydroxyl groups which helps attaching the gauze to the layer(s) comprising nanofibrillar cellulose via hydrogen bonds. Also semi-synthetic fibers may provide free hydroxyl groups, such as viscose.

In one embodiment the gauze comprises natural gauze, such as cellulose or cotton gauze, synthetic gauze or semi-synthetic gauze, or a mixture thereof. In one example the gauze comprise a mixture of polypropylene and cellulose. In one example the gauze comprise a mixture of polypropylene, polyester and cellulose. In one example the gauze comprise a mixture of viscose and polypropylene. In one example the gauze comprise a mixture of viscose and polyester. Cellulose fibers may be mixed with these materials. These gauzes may be non-woven.

The gauze should be highly permeable allowing fluids to pass through. The gauze is not a filter and it does not limit the flow through of most macromolecules. The gauze may not be used as a filter for dewatering a dispersion comprising nanofibrillar cellulose. The gauze may be porous and/or it may be fenestarated having perforations or slits or the like. A paper or cardboard is not a gauze. More particularly paper is not suitable as paper does not provide high enough tear strength in such grammages or thicknesses which would be suitable for the multi-layer products. The same applies to cardboard or other similar cellulosic products. In one example the gauze is non-cellulosic.

In one example the gauze is resilient. Many natural, semi-synthetic or synthetic fibers are resilient. However, in one example the gauze is rigid providing non-resilient properties, for example when it comprises cotton. The gauze may provide reinforcing properties, for example to enhance the tear strength of the multi-layer product.

Tear strength (tear resistance) is a measure of how well a material can withstand the effects of tearing. More specifically it measures how well a material resists the growth of any cuts when under tension. Tear resistance may be measured by the ASTM D 412 method (the same may be used to measure tensile strength, modulus and elongation). Also a tear index may be presented, wherein tear index = tear strength/grammage, and it is usually measured in mNm²/g.

The gauze may have a tear strength in the range of 800-2000 mN. Tear index may be measured with ISO 1974. The tensile strength of a gauze may be for example in the range of 0.6-1.5 kN/m, such as 0.7-1.2 kN/m. Tensile strength may be measured by ISO 1924-3. The gauze may have a grammage in the range of 20-60 g/m², for example in the range of 30-55 g/m². Grammage may be measured by ISO 536. The gauze may have a density for example in the range of 100-400 g/cm³, such as in the range of 160-330 g/cm³. Also a bulk may be presented as cm³/g, measured by ISO 534.

A layer of gauze, such as a dry gauze, may have a thickness in the range of 100-1000 µm, such as 100-200 µm, 150-200 µm, 200-300 µm, 300-400 µm, 400-500 µm, 500-600 µm, 600-700 µm, 700-800 µm, 800-900 µm or 900-1000 µm. However, thicker gauzes may also be used, for example up to 2000 or 3000 µm. In one example the thickness of the gauze is in the range of 100-200 µm, such as 100-120 µm, 120-140 µm, or 140-160 µm or 160-190 µm.

One embodiment provides a medical product comprising a layer of gauze impregnated with nanofibrillar cellulose so that the nanofibrillar cellulose is distributed evenly in the gauze and no separate layers are formed from the nanofibrillar cellulose on any surface of the gauze. In one embodiment the medical product is obtained with a method described herein comprising pressing the impregnated gauze before dewatering in a nip, such as in a nip roll and/or wherein the impregnating and dewatering are repeated until the medical product has reached a grammage in the range of 25-80 g/m².

The medical product may have a thickness in the range of 50-500 µm. In one example the medical product has a thickness in the range of 50-250 µm, such as 80-200 µm, or 100-150 µm, or 110-140 µm. Thickness may be measured as bulking thickness by ISO 534.

With a reinforcing gauze the tear strength of the medical structure is remarkably higher than in a product without the gauze. In one example the medical product has a tear index in the range of 10-100 mNm²/g. In one example the medical product has a tear index in the range of 15-70 mNm²/g. The tear strength may be different in one direction and in a perpendicular direction, which may be affected by the properties of the gauze. For example a gauze may have different properties to the perpendicular directions, which may be called as machine direction (md) and cross direction (cd).

In one embodiment the medical product has a grammage in the range of 25-80 g/m². In one embodiment the medical product has a grammage in the range of 30-70 g/m². In one embodiment the medical product has a grammage in the range of 35-65 g/m². In one example the medical product has a grammage in the range of 45-63 g/m².

The grammage of the nanofibrillar cellulose in the medical product may be in the range of 1-50 g/m², for example 1-20 g/m², such as 2-20 g/m², 2-12 g/m² or 5-15 g/m², measured as dry weight of the product.

In one example the medical product has a density in the range of 300-700 g/cm³, such as 350-530 kg/m³. The density may be measured as apparent bulking density by ISO 534.

The air permeance of the medical product, preferably as autoclaved, may be less than 120 ml/min, or less than 600 ml/min, such as less than 1000 ml/min or less than 2000 ml/min. The air permeance correlates in general with the amount of nanofibrillar cellulose. The higher the amount of nanofibrillar cellulose, the lower the air permeance. With an exemplary air permeance of less than 600 ml/min, or less than 500 ml/min the amount of nanocellulose is at suitable level for many applications.

The medical products may be used in several applications. One specific field is medical applications, wherein the materials are applied on living tissue, such as skin. The structures may be used in medical products, such as patches, dressings, bandages, filters and the like. The medical products may also be therapeutic products, such as therapeutic patches containing medicament. In general the surface of the product comprising nanofibrillar cellulose will be in contact with the skin during the use. A surface of nanofibrillar cellulose may provide advantageous effects when it is in direct contact with the skin, for example it may promote healing of a wound or other damage on a skin, or it may promote delivery of substances from the medical product to the skin.

The term "wound" as used herein refers to any damages, injuries, diseases, disorders or the like on a tissue, such as skin, including open or closed wounds, wherein the healing of the wound is desired and may be promoted with the product described herein. The wound may be clean, contaminated, infected or colonized, wherein especially in the latter cases a therapeutic agent, such as an antibiotic, may be administered. Examples of open wounds include abrasions, avulsions, incisions, lacerations, puncture wounds and penetration wounds. Examples of closed wounds include hematomas, crush injuries, sewn wounds, grafts and any skin conditions, diseases or disorders.

Examples of conditions, diseases or disorders of the skin include acne, infections, vesiculobullous diseases, cold sore, cutaneous candidiasis, cellulitis, dermatitis and eczema, herpes, hives, lupus, papulosquamous, urticaria and erythema, psoriasis, rosacea, radiation-related disorders, pigmentation, mucinoses keratosis, ulcer, atrophy, and necrobiosis, vasculitis, vitiligo, warts, neutrophilic and eosinophilic diseases, congenital, neoplasms and cancer, such as melanomas and tumours of epidermis or dermis, or other diseases or disorders of epidermis and dermis.

A medical product comprising a therapeutic agent may be provided, wherein the gauze and/or the surface layer comprising nanofibrillar cellulose contain(s) one or more therapeutic agent, such as a medicament or drug. Also the term pharmaceutical agent may be used interchangeably instead of the term therapeutic agent. Such agents are active or effective agents, which are usually present in effective amounts. Such an agent may be provided in a predetermined amount, for example in an amount configured to provide a desired dose of the agent during a certain time period, and/or configured to provide a desired effect on the target, such as skin or other tissue. The content of the therapeutic agent in the product may be for example in the range of 0.1-5%. Especially if the therapeutic agent is included, a sustained or prolonged release of the agent may be provided. In such case the nanofibrillar cellulose may contain a portion of moisture to enable permeability of the agent. The moisture content of the product comprising therapeutic agent may be in the range of 0-10%, such as in the range of 5-7%. The therapeutic agents may be present in water-soluble form, fat-soluble form or in an emulsion, or in another suitable form.

Examples of therapeutic agents which may be administered by using the medical products described herein include antibiotics, pain relievers, such as lidocaine; nicotine; opioids, such as fentanyl or buprenorphine; hormones, such as estrogen, contraceptives or testosterone; nitroglycerin; scopolamine; clonidine; antidepressants, such as selegiline; ADHD medication, such as methylphenidate; vitamins, such as B12 or cyanocobalamin; 5-hydroxytryptophan; Alzheimer's medication, such as rivastigmine; acne medication; antipsoriatics, glucocorticoids such as hydrocortisone; or any other medication for treating diseases or disorders of a skin. Therapeutic agents may be used for example in medical patches, which may be used on healthy skin or on damaged skin, to provide a prolonged, sustained or extended release of the therapeutic agent from the patch, for example during a period of several hours, for up to 6, 12, 24 or even 48 hours.

One example provides the medical product comprising antibiotic agent. Such a product is especially suitable for treating wounds, wherein the wound treating properties are combined with antibiotic properties which prevents infections caused by harmful microbes in the wound. Examples of suitable antibiotics include especially topical antibiotics, such as bacitracin, erythromycin, clindamycin, gentamycin, neomycin, polymyxin, mupirocin, tetracycline, meclocycline, (sodium) sulfacetamide, benzoyl peroxide, and azelaic acid, and combinations thereof. Also other types of antibiotics, such as systemic antibiotics, may be provided, for example penicillins, such as phenoxymethylpenicillin, flucloxacillin and amoxicillin; cephalosporins, such as cefaclor, cefadroxil and cephalexin; tetracyclines, such as tetracycline, doxycycline and lymecycline; aminoglycosides, such as gentamicin and tobramycin; macrolides, such as erythromycin, azithromycin and clarithromycin; clindamycin; sulphonamides and trimethoprim; metronidazole and tinidazole; quinolones, such as ciprofloxacin, levofloxacin and norfloxacin.

Antibiotics may be also used for treating acne, for example clindamycin, erythromycin, doxycycline, tetracycline etc. Also other agents may be used, such as benzoyl peroxide, salicylic acid, topical retinoid medicines, such as tretinoin, adapalene or tazarotene, azelaic acid, or androgen blockers such as spirolactone. Psoriasis may be treated for example with steroids, such as corticosteroids, moisturizers, calciprotriene, coal tar, vitamin D, retinoids, tazatorene, anthralin, salisylic acid, methotrexate, or cyclosporine. Insect bites or poison ivy exposure may be treated with agents such as hydrocortisone, emu oil, almond oil, ammonia, bisabolol, papain, diphenylhydramine, jewelweed axtract or calamine. Some of these or other treatment agents may be also categorized as cosmetic agents.

One embodiment provides a medical product, such as a dressing, a patch or a filter, comprising the impregnated medical product described herein.

One embodiment provides the medical product for use for treating and/or covering skin wounds or other damages. One example provides such a medical product for use as a dressing or a patch, or in a dressing or a patch, for treating and/or covering skin wounds or other damages.

One embodiment provides such a medical product for use for treating and/or covering skin wounds covered with a graft, such as a skin graft. One example provides such a medical product for use as a dressing or a patch, or in a dressing or a patch, for treating and/or covering skin wounds covered with a graft, such as a skin graft.

A dressing is a sterile pad or compress applied to a wound to promote healing and/or prevent further harm. A dressing is designed to be in direct contact with the wound, as distinguished from a bandage, which is most often used to hold a dressing in place. Some organizations classify them as the same thing (for example, the British Pharmacopoeia) and the terms are used interchangeably by some people. Dressings are frequently used in first aid and nursing.

One embodiment provides the medical product for use for administering therapeutic agent. In such case the medical product may be provided as such or for example in a patch. One or more therapeutic agent(s) may be included, for example impregnated, in the product as described herein, and the administration to a patient may be dermal or transdermal.

One embodiment provides a cosmetic product, such as a dressing, a mask or a patch, comprising the medical product. Such a product may be called also as a cosmetic product. The product may be provided in various shapes, for example a mask may be designed to fit onto face, for example below eye or onto chin, nose or forehead. One example provides the medical product for use as a cosmetic product. The product may be used for releasing one or more cosmetic agent(s) to the user, such as to the skin of the user. Such a cosmetic product may comprise one or more cosmetic agent(s). Cosmetic agent(s) may be included, for example impregnated, in the product wherefrom they will be released or delivered. The content of a cosmetic agent in the product may be for example in the range of 0.1-5%. The cosmetic agents may be present or provided in the product similarly as explained above for therapeutic agents, and vice versa. The cosmetic use may be analogous to medical use described herein, especially the administering of therapeutic agent. Cosmetic agents may be used also for cosmetically treating skin diseases or disorders, such as those mentioned herein. Such cosmetic products may be used for example for treating pimples, acneic skin, brown sports, wrinkles, oily skin, dry skin, aged skin, spider veins, after sun erythemas, black circles etc. Examples of cosmetic patches include skin cleansers, such as pore cleansers, blackhead removers, stretching stripes, short-term patch-like masks, short-term treatment patches and overnight treatment patches.

Examples of cosmetic agents include forms of vitamins and precursors thereof, such as vitamin A; for example retinoids, such as retinaldehyde (retinal), retinoic acid, retinyl palmitate and retinyl retinoate, ascorbic acid, alpha-hydroxy acids such as glycolic acid and lactic acid; glycols; biotechnology products; keratolytics; amino acids; antimicrobials; moisturizers; pigments; antioxidants; plant extracts; cleansing agents or make-up removers; anti-cellulite agents such as caffeine, carnitine, ginkgo biloba and horse-chestnut; conditioners; fragrances such as aromatherapy agents and perfumes; humectants such as urea, hyaluronic acid, lactic acid and glycerine; emollients such as lanolin, triglycerides and fatty acid esters; FR scavengers, singlet oxygen scavengers, superoxide scavengers or hydrogen peroxide scavengers, such as ascorbic acid (vitamin C), glutathione, tocopherol (vitamin E), carotenoids, coenzyme Q10, bilirubin, lipoic acid, uric acid, enzyme mimetic agents, idebenone, polyphenols, selenium, spin traps such as phenyl butyl nitrone (PBN), protein methionine groups, superoxide dismutase, catalase, selenium peroxidases, heme oxygenases etc. or combinations thereof. The cosmetic agents may be present in water-soluble form, fat-soluble form or in an emulsion, or in another suitable form.

One example useful for understanding the invention provides a method for cosmetically treating skin, the method comprising applying the medical product described herein onto skin.

A "patch" as used herein refers to a medical or cosmetic product which may be applied onto skin. Examples of patches include dermal patch and transdermal patch. A dermal patch or skin patch is a medicated adhesive patch that is placed on the skin to deliver a medication into the skin. A transdermal patch is a medicated adhesive patch that is applied on the skin to deliver a specific dose of medication through the skin and into the bloodstream. In one example this promotes healing to an injured area of the body. A patch may contain a release liner, which protects the patch during storage and is removed prior to use, and/or adhesive for adhering the patch to the skin, and/or backing for protecting the patch from the outer environment. Examples of release liners include paper-based liners, such as glassine paper, densified Kraft super-calendered paper, clay-coated paper, silicone-coated paper and polyolefine-coated paper; plastic based liner, such as polystyrene, polyester, polyethylene, cast polypropylene and polyvinyl chloride; and composite material liners based on the combination of several films. Adhesive layers may contain for example pressure sensitive adhesive (PSA).

One example provides the medical product described herein packed in a separate packing. Separate packings may be provided as a series of packings. Usually such packed products are provided as sterilized.

One example provides a kit comprising the medical product or the cosmetic product described herein, for example a packed product, wherein the kit may contain one or more of the packed products. The kit may also contain other materials or equipment, such as a container containing saline solution or the like for pretreating the product(s) prior to use.

One example useful for understanding the invention provides the medical product for use in a method for treating skin wounds or other damages or injuries, the method comprising applying the medical product described herein onto the wound, damage, or injury. One specific example useful for understanding the invention provides the medical product for use in a method for treating skin wounds covered with a graft, such as a skin graft, for example a mesh graft or a full thickness graft, the method comprising applying the medical product described herein onto the graft.

Grafting refers to a surgical procedure to move tissue from one site to another on the body, or from another person, without bringing its own blood supply with it. Instead, a new blood supply grows in after it is placed. Autografts and isografts are usually not considered as foreign and, therefore, do not elicit rejection. Allografts and xenografts are recognized as foreign by the recipient and are rejected.

Skin grafting is often used to treat skin loss due to a wound, burn, infection, or surgery. In the case of damaged skin, it is removed, and new skin is grafted in its place. Skin grafting can reduce the course of treatment and hospitalization needed, and can also improve function and appearance. There are two types of skin grafts: Split-thickness skin grafts (epidermis + part of the dermis) and full-thickness skin grafts (epidermis + entire thickness of the dermis).

A mesh graft is a full- or partial-thickness sheet of skin that has been fenestrated to allow drainage and expansion. Mesh grafts are useful in many locations on the body because they conform to uneven surfaces. They can be placed in locations that have excessive motion because they can be sutured to the underlying wound bed. Additionally, their fenestrations provide outlets for fluid that may accumulate beneath the graft, which helps reduce tension and the risk of infection and improve vascularization of the graft.

It was found out in the clinical tests that the medical product attaches to a graft area and acts as a protective layer. As the graft heals, the product forms a scab-like structure together with the graft. The properties of the product comprising nanofibrillar cellulose promote the healing, and the medical product with the formed dry scab will come loose in similar way as a regular scab behaves in normal wound healing process.

Before applying the medical product onto skin the product may be pretreated i.e. moisture or wetted, in general with an aqueous solution. The moisturizing or wetting may be carried out for example by using water or regular physiological saline solution, which is usually a solution of 0.90% w/w of NaCl, having an osmolality of about 308 mOsm/l. Other types of aqueous solutions may also be used, such as saline solutions with different concentrations. Moisturizing or wetting the material enhances contact with the skin and the moldability of a sheet of material.

### Examples

### Example 1

Table 1 shows results from different impregnation tests with different gauzes. The tests AE5-AE7 were run by one Imprex nip. In AE9 there were 3 nips and in AE10 2 nips. The tests AH1-AH5 were run with one Imprex nip with 3, 4, 4, 5, and 4 impregnation runs respectively. The drying temperature was 90°C in all the tests. In tests AE5-AE10 the drying pressure was 2 bars and in the tests AH1-AH5 the drying pressure was 5 bars. The drying was carried out in a press dryer between a Teflon plate and a cloth. The gel consistency was 0.8% in all the tests. An impregnation run includes impregnation, pressing in the nip and dewatering.

The gauzes used were pulp-polypropylene-polyester gauzes (AE7, AE9, AE10, AH3, AH4), pulp-polypropylene-polyester gauzes (AE5), pulp-polypropylene gauze (AH1, AH2), pulp-based gauze (AH5).

Properties from the starting materials and the obtained aerated products were measured, such as grammages (g/cm²) from the final product, gauze and nanofibrillar cellulose (NFC), average thickness (µm), density (g/cm³), roughness (Bendtsen, ml/min) from aerated and from autoclaved and aerated products from upper and lower sides, and air permeance (Bentdsen, ml/min) from aerated products. The properties were measured according to ISO standards mentioned in the specification where applicable.

In general it was observed that in one impregnation run (IR) about 1.5 g/m² of nanofibrillar cellulose was transferred, but this however depends on the gauze. There was also great variance in the grammages of the gauzes even in the same batch

**Table 1. Results from different impregnation tests with different gauzes.**

| No | IR | g/m² g/m² | Gauze | NFC g/m² | Thickne ss avg µm | g/cm³ | Rough. upper ml/min | Rough. lower ml/min | Rough. autocl. upper ml/min | Rough. autocl. lower ml/min | Air permeance ml/min | Air permeance autoclaved ml/min |
|---|---|---|---|---|---|---|---|---|---|---|---|---|
| AE5 | 1 | 44 | 43 | 1 | 128 | 359 | | | | | 8820 | |
| AE7 | 1 | 61 | | | 147 | 390 | | | | | 8820 | |
| AE9 | 3 | 58 | 55 | 3 | 135 | 421 | | | | | 1770 | |
| AE10 | 2 | 57 | 55 | 2 | 116 | 483 | | | | | 3370 | |
| AH1 | 3 | 52 | 47 | 5 | 114 | 452 | 1675 | 1623 | 2824 | 2208 | 2275 | 2580 |
| AH2 | 4 | 52 | 47 | 5 | 109 | 489 | 1818 | 391 | 2276 | 863 | 652 | 1360 |
| AH3 | 4 | 63 | 55 | 8 | 115 | 530 | 1338 | 432 | 1483 | 669 | 123 | 157 |
| AH4 | 5 | 62 | 55 | 7 | 115 | 530 | 645 | 719 | 809 | 751 | 94 | 131 |
| AH5 | 4 | 43 | 38 | 4 | 82 | 515 | 565 | 518 | 900 | 559 | 90 | 181 |

### Example 2

The effect of the number of impregnation runs (impregnation passes including impregnation, pressing in a nip and dewatering) to the air permeance of the product was tested (Table 2). The gauzes were as explained in the Example 1.

**Table 2. Porosity vs. impregnation passes**

| No | Bendtsen air permeance, no air conditioning (ml/min) | | | | |
|---|---|---|---|---|---|
| IR | 1 | 2 | 3 | 4 | 5 |
| AH4 | | | 721 | 367 | 79 |
| AH5 | 7393 | 2580 | 673 | 298 | 99 |
| AE 7/10/9 | 8820 | 3370 | 1770 | | |

### Example 3

Properties of different medical products prepared from different gauzes were tested. AJ1, AJ2, AK2 and AK3 are pulp-polypropylene gauzes. AK1 is pulp-polypropylene-polyester gauze. Grammages and air permeances were measured from air conditioned samples. The results are presented in Table 3. Md = machine direction, cd = cross direction.

**Table 3. Properties of the medical products**

| **No** | **IR** | **g/m²** | **Gauze g/m²** | **NFC g/m²** | **Thickn ess avg µm** | **g/cm³** | **Air permeanc e Bendtsen ml/min** | **Tear strength md mN** | **Tear strength cd mN** | **Tensile strength md kN/m** | **Tensile strength cd kN/m** |
|---|---|---|---|---|---|---|---|---|---|---|---|
| AJ1 | 5 | 55 | 47 | 7 | 128 | 428 | 1935 | | | 1.3 | |
| AJ2 | 4 | 53 | 47 | 6 | 131 | 407 | 3785 | 956 | 1635 | 1.1 | |
| AK1 | 5 | 47 | 43 | 4 | 112 | 420 | 311 | 1528 | 2088 | | 0.4 |
| AK2 | 5 | 51 | 47 | 4 | 109 | 473 | 596 | | | 1.3 | |
| AK3 | 5 | 51 | 47 | 4 | 108 | 473 | 644.5 | | | 1.5 | 0.5 |

### Example 4

Properties of different gauzes HA1-HA7 were analyzed. Grammages were measured from air conditioned samples. The results are presented in Tables 4 and 5. Md = machine direction, cd = cross direction.

**Table 4. Gauze properties**

| **No** | **Gauze material** | **g/m²** | **Thicknes s avg µm** | **g/cm³** | **Break elongatio n, md %** | Tear strength, md mN | Tear strength cd mN | Tensile strength md kN/m |
|---|---|---|---|---|---|---|---|---|
| HA1 | Pulp-Polypropylene | 47 | 260 | 179 | 27.2 | 884 | 1859 | 1.0 |
| HA2 | Pulp-Polypropylene | 39 | 229 | 169 | 24.35 | 1143 | 1258 | 0.9 |
| HA3 | Pulp-Polypropylene - Polyester | 45 | 140 | 321 | | 1879 | | |
| HA4 | Pulp-Polypropylene - Polyester | 50 | 259 | 193 | | 1856 | | |
| HA5 | Polyester-Viscose | 33 | 105 | 307 | | 1794 | | 1.1 |
| HA6 | Pulp | 39 | 187 | 206 | | | | |
| HA7 | Cotton | 32 | 179 | 178 | | | | |

**Table 5. Gauze properties**

| No | Gauze material | Tear index md **mNm²/g** | Tear index cd **mNm²/g** | Tensile index md Nm/g |
|---|---|---|---|---|
| HA1 | Pulp-Polypropylene | 19 | 40 | 21 |
| HA2 | Pulp-Polypropylene | 30 | 33 | 24 |
| HA3 | Pulp-Polypropylene-Polyester | 42 | | |
| HA4 | Pulp-Polypropylene-Polyester | 37 | | |
| HA5 | Polyester-Viscose | 55 | | |
| HA6 | Pulp | | | |
| HA7 | Cotton | | | |

## Claims

1. A method for preparing a medical product, said method comprising
- providing an aqueous dispersion of nanofibrillar cellulose,
- providing a layer of gauze,
- impregnating the layer of gauze by soaking throughout with the aqueous dispersion of nanofibrillar cellulose so that the nanofibrillar cellulose is distributed evenly in the gauze and no separate layers are formed from the nanofibrillar cellulose on any surface of the gauze, and
- dewatering the impregnated gauze,
to obtain the medical product.

2. The method of claim 1, comprising pressing the impregnated gauze before dewatering in a nip, such as in a nip roll.

3. The method of any of the preceding claims, wherein the impregnating and dewatering are repeated until the medical product has reached a grammage in the range of 25-80 g/m², such as 30-70 g/m², for example 35-65 g/m².

4. The method of any of the preceding claims, wherein the gauze comprises natural gauze, such as cotton gauze, synthetic gauze or semi-synthetic gauze, such as viscose, polypropylene or polyester gauze, or a mixture thereof.

5. The method of any of the preceding claims, wherein the nanofibrillar cellulose, when dispersed in water, provides a Brookfield viscosity of at least 2000 mPa·s, such as at least 3000 mPa·s, for example at least 10000 mPa·s, measured at a consistency of 0.8% (w/w) and at 10 rpm, and/or wherein the nanofibrillar cellulose has a number average diameter of a fibril in the range of 1-100 nm.

6. The method of any of the preceding claims, wherein the nanofibrillar cellulose comprises non-modified nanofibrillar cellulose.

7. The method of any of the preceding claims, wherein the nanofibrillar cellulose comprises chemically modified nanofibrillar cellulose, for example anionically modified nanofibrillar cellulose, such as oxidized nanofibrillar cellulose, or enzymatically modified nanofibrillar cellulose.

8. The method of any of the preceding claims, wherein the dewatering is carried out by non-contact drying, such as with an infrared dryer, floating dryer, or impingement dryer, or by contact drying, such as with a press dryer, cylinder dryer or belt dryer.

9. A dewatered medical product comprising a layer of gauze impregnated thoroughly with nanofibrillar cellulose so that the nanofibrillar cellulose is distributed evenly in the gauze and no separate layers are formed from the nanofibrillar cellulose on any surface of the gauze.

10. The medical product of claim 9,
wherein the gauze comprises natural gauze, such as cellulose or cotton gauze, synthetic gauze or semi-synthetic gauze, such as viscose or polyester, or a mixture thereof, for example a mixture of polypropylene and cellulose or a mixture of polypropylene, polyester and cellulose, and/or wherein the gauze is nonwoven.

11. The medical product of claim 9 or 10, wherein the nanofibrillar cellulose, when dispersed in water, provides a Brookfield viscosity of at least 2000 mPa·s, such as at least 3000 mPa·s, for example at least 10000 mPa·s, measured at a consistency of 0.8% (w/w) and at 10 rpm, and/or wherein the nanofibrillar cellulose has a number average diameter of a fibril in the range of 1-100 nm.

12. The medical product of any of the claims 9-11, wherein the nanofibrillar cellulose comprises non-modified nanofibrillar cellulose.

13. The medical product of any of the claims 9-12, wherein the nanofibrillar cellulose comprises chemically modified nanofibrillar cellulose, for example anionically modified nanofibrillar cellulose, such as oxidized nanofibrillar cellulose, or enzymatically modified nanofibrillar cellulose.

14. The medical product of any of the claims 9-13, wherein product has a grammage in the range of 25-80 g/m², such as 30-70 g/m², for example 35-65 g/m².

15. The medical product of any of the claims 9-14 obtained with the method of claim 2 or 3.

16. The medical product of any of the claims 9-15 comprising a therapeutic agent.

17. The medical product of any of the claims 9-16 comprising a cosmetic agent.

18. A medical dressing or a patch, comprising the medical product of any of the claims 9-17.

19. The medical product of any of the claims 9-17 for use in treating and/or covering skin wounds or other damages, such as skin wounds covered with a graft, for example a skin graft.

20. The medical product of any of the claims 9-17 for use in administering a therapeutic agent.

21. A cosmetic product, such as a dressing or a patch, comprising the medical product of any of the claims 9-15 or 17.

## Patentansprüche

1. Verfahren zur Herstellung eines medizinischen Produkts, wobei das Verfahren umfasst:
- Bereitstellen einer wässrigen Dispersion von nanofibrillärer Cellulose,
- Bereitstellen einer Mullschicht,
- Imprägnieren der Mullschicht durch vollständiges Tränken mit der wässrigen Dispersion von nanofibrillärer Cellulose, so dass die nanofibrilläre Cellulose gleichmäßig in dem Mull verteilt wird und keine separaten Schichten von der nanofibrillären Cellulose auf einer beliebigen Oberfläche des Mulls gebildet werden, und
- Entwässern des imprägnierten Mulls,
um das medizinische Produkt zu erhalten.

2. Verfahren nach Anspruch 1, umfassend ein Pressen des imprägnierten Mulls in einem Walzenspalt, wie in einer Druckwalze, vor dem Entwässern.

3. Verfahren nach einem der vorhergehenden Ansprüche, wobei das Imprägnieren und das Entwässern wiederholt werden, bis das medizinische Produkt eine Grammatur im Bereich von 25-80 g/m², wie 30-70 g/m², beispielsweise 35-65 g/m², erreicht hat.

4. Verfahren nach einem der vorhergehenden Ansprüche, wobei der Mull natürlichen Mull, wie Baumwollmull, synthetischen Mull oder halbsynthetischen Mull, wie Viskose-, Polypropylen- oder Polyestermull, oder ein Gemisch davon umfasst.

5. Verfahren nach einem der vorhergehenden Ansprüche, wobei die nanofibrilläre Cellulose, wenn sie in Wasser dispergiert wird, eine Brookfield-Viskosität von mindestens 2000 mPa·s, wie mindestens 3000 mPa·s, beispielsweise mindestens 10.000 mPa·s, gemessen bei einer Konsistenz von 0,8 % (w/w) und bei 10 U/min, bereitstellt und/oder wobei die nanofibrilläre Cellulose einen zahlenmittleren Durchmesser einer Fibrille im Bereich von 1-100 nm aufweist.

6. Verfahren nach einem der vorhergehenden Ansprüche, wobei die nanofibrilläre Cellulose unmodifizierte nanofibrilläre Cellulose umfasst.

7. Verfahren nach einem der vorhergehenden Ansprüche, wobei die nanofibrilläre Cellulose chemisch modifizierte nanofibrilläre Cellulose, beispielsweise anionisch modifizierte nanofibrilläre Cellulose, wie oxidierte nanofibrilläre Cellulose, oder enzymatisch modifizierte nanofibrilläre Cellulose umfasst.

8. Verfahren nach einem der vorhergehenden Ansprüche, wobei das Entwässern durch kontaktloses Trocknen, wie mit einem Infrarottrockner, Schwebetrockner oder Pralltrockner, oder Kontakttrocknen, wie mit einem Pressentrockner, Zylindertrockner oder Bandtrockner, durchgeführt wird.

9. Entwässertes medizinisches Produkt, umfassend eine Mullschicht, die vollständig mit nanofibrillärer Cellulose imprägniert ist, so dass die nanofibrilläre Cellulose gleichmäßig in dem Mull verteilt wird und keine separaten Schichten von der nanofibrillären Cellulose auf einer beliebigen Oberfläche des Mulls gebildet werden.

10. Medizinisches Produkt nach Anspruch 9, wobei der Mull natürlichen Mull, wie Cellulose- oder Baumwollmull, synthetischen Mull oder halbsynthetischen Mull, wie Viskose oder Polyester, oder ein Gemisch davon, beispielsweise ein Gemisch von Polypropylen und Cellulose oder ein Gemisch von Polypropylen, Polyester und Cellulose, umfasst und/oder wobei der Mull ein Vliesstoff ist.

11. Medizinisches Produkt nach Anspruch 9 oder 10, wobei die nanofibrilläre Cellulose, wenn sie in Wasser dispergiert wird, eine Brookfield-Viskosität von mindestens 2000 mPa·s, wie mindestens 3000 mPa·s, beispielsweise mindestens 10.000 mPa·s, gemessen bei einer Konsistenz von 0,8 % (w/w) und bei 10 U/min, bereitstellt und/oder wobei die nanofibrilläre Cellulose einen zahlenmittleren Durchmesser einer Fibrille im Bereich von 1-100 nm aufweist.

12. Medizinisches Produkt nach einem der Ansprüche 9-11, wobei die nanofibrilläre Cellulose unmodifizierte nanofibrilläre Cellulose umfasst.

13. Medizinisches Produkt nach einem der Ansprüche 9-12, wobei die nanofibrilläre Cellulose chemisch modifizierte nanofibrilläre Cellulose, beispielsweise anionisch modifizierte nanofibrilläre Cellulose, wie oxidierte nanofibrilläre Cellulose, oder enzymatisch modifizierte nanofibrilläre Cellulose umfasst.

14. Medizinisches Produkt nach einem der Ansprüche 9-13, wobei das Produkt eine Grammatur im Bereich von 25-80 g/m², wie 30-70 g/m², beispielsweise 35-65 g/m², aufweist.

15. Medizinisches Produkt nach einem der Ansprüche 9-14, erhalten mit dem Verfahren nach Anspruch 2 oder 3.

16. Medizinisches Produkt nach einem der Ansprüche 9-15, umfassend ein Therapeutikum.

17. Medizinisches Produkt nach einem der Ansprüche 9-16, umfassend ein Kosmetikum.

18. Medizinisches Verbandmaterial oder Pflaster, umfassend das medizinische Produkt nach einem der Ansprüche 9-17.

19. Medizinisches Produkt nach einem der Ansprüche 9-17 zur Verwendung beim Behandeln und/oder Abdecken von Hautwunden oder anderen Verletzungen, wie Hautwunden, die mit einem Transplantat, beispielsweise einem Hauttransplantat, bedeckt sind.

20. Medizinisches Produkt nach einem der Ansprüche 9-17 zur Verwendung beim Verabreichen eines Therapeutikums.

21. Kosmetikprodukt, wie ein Verbandmaterial oder Pflaster, umfassend das medizinische Produkt nach einem der Ansprüche 9-15 oder 17.

## Revendications

1. Procédé de préparation d'un produit médical, ledit procédé comprenant
- fournir une dispersion aqueuse de cellulose nanofibrillaire,
- fournir une couche de gaze,
- imprégner la couche de gaze en la trempant entièrement avec la dispersion aqueuse de cellulose nanofibrillaire de sorte que la cellulose nanofibrillaire soit répartie uniformément dans la gaze et qu'aucune couche séparée ne soit formée à partir de la cellulose nanofibrillaire sur aucune surface de la gaze, et
- déshydrater la gaze imprégnée,
pour obtenir le produit médical.

2. Procédé de la revendication 1, comprenant le pressage de la gaze imprégnée avant la déshydratation dans un pincement, par exemple dans un rouleau pinceur.

3. Procédé de l'une des revendications précédentes, dans lequel l'imprégnation et la déshydratation sont répétées jusqu'à ce que le produit médical ait atteint un grammage dans la plage allant de 25 à 80 g/m², tel que 30 à 70 g/m², par exemple 35 à 65 g/m².

4. Procédé de l'une des revendications précédentes, dans lequel la gaze comprend une gaze naturelle, telle qu'une gaze de coton, une gaze synthétique ou une gaze semi-synthétique, telle qu'une gaze de viscose, de polypropylène ou de polyester, ou un mélange de celles-ci.

5. Procédé de l'une des revendications précédentes, dans lequel la cellulose nanofibrillaire, lorsqu'elle est dispersée dans l'eau, fournit une viscosité Brookfield d'au moins 2000 mPa.s, telle que d'au moins 3000 mPa.s, par exemple d'au moins 10000 mPa.s, mesurée à une consistance de 0,8% (p/p) et à 10 tr/min, et/ou dans lequel la cellulose nanofibrillaire a un diamètre moyen en nombre d'une fibrille dans la plage allant de 1 à 100 nm.

6. Procédé de l'une des revendications précédentes, dans lequel la cellulose nanofibrillaire comprend une cellulose nanofibrillaire non modifiée.

7. Procédé de l'une des revendications précédentes, dans lequel la cellulose nanofibrillaire comprend une cellulose nanofibrillaire modifiée chimiquement, par exemple une cellulose nanofibrillaire modifiée anioniquement, telle qu'une cellulose nanofibrillaire oxydée, ou une cellulose nanofibrillaire modifiée enzymatiquement.

8. Procédé de l'une des revendications précédentes, dans lequel la déshydratation est effectuée par séchage sans contact, tel qu'avec un sécheur infrarouge, un sécheur flottant ou un sécheur à impact, ou par séchage par contact, tel qu'avec un sécheur à presse, un sécheur à cylindres ou un sécheur à bande.

9. Produit médical déshydraté comprenant une couche de gaze complètement imprégnée de cellulose nanofibrillaire de sorte que la cellulose nanofibrillaire soit répartie uniformément dans la gaze et qu'aucune couche séparée ne soit formée à partir de la cellulose nanofibrillaire sur aucune surface de la gaze.

10. Produit médical de la revendication 9, dans lequel la gaze comprend une gaze naturelle, telle qu'une gaze de cellulose ou de coton, une gaze synthétique ou une gaze semi-synthétique, telle que de la viscose ou du polyester, ou un mélange de celles-ci, par exemple un mélange de polypropylène et de cellulose ou un mélange de polypropylène, de polyester et de cellulose, et/ou dans lequel la gaze est non tissée.

11. Produit médical de la revendication 9 ou 10, dans lequel la cellulose nanofibrillaire, lorsqu'elle est dispersée dans l'eau, fournit une viscosité Brookfield d'au moins 2000 mPa.s, telle que d'au moins 3000 mPa.s, par exemple d'au moins 10000 mPa.s, mesurée à une consistance de 0,8% (p/p) et à 10 tr/min, et/ou dans lequel la cellulose nanofibrillaire a un diamètre moyen en nombre d'une fibrille dans la plage allant de 1 à 100 nm.

12. Produit médical de l'une des revendications 9 à 11, dans lequel la cellulose nanofibrillaire comprend une cellulose nanofibrillaire non modifiée.

13. Produit médical de l'une des revendications 9 à 12, dans lequel la cellulose nanofibrillaire comprend une cellulose nanofibrillaire modifiée chimiquement, par exemple une cellulose nanofibrillaire modifiée anioniquement, telle qu'une cellulose nanofibrillaire oxydée, ou une cellulose nanofibrillaire modifiée enzymatiquement.

14. Produit médical de l'une des revendications 9 à 13, dans lequel le produit a un grammage dans la plage allant de 25 à 80 g/m², tel que 30 à 70 g/m², par exemple 35 à 65 g/m².

15. Produit médical de l'une des revendications 9 à 14 obtenu avec le procédé de la revendication 2 ou 3.

16. Produit médical de l'une des revendications 9 à 15 comprenant un agent thérapeutique.

17. Produit médical de l'une des revendications 9 à 16 comprenant un agent cosmétique.

18. Pansement médical ou patch, comprenant le produit médical de l'une des revendications 9 à 17.

19. Produit médical de l'une des revendications 9 à 17 pour une utilisation dans le traitement et/ou le recouvrement de plaies cutanées ou d'autres dommages, tels que des plaies cutanées recouvertes d'une greffe, par exemple d'une greffe de peau.

20. Produit médical de l'une des revendications 9 à 17 pour une utilisation dans l'administration d'un agent thérapeutique.

21. Produit cosmétique, tel qu'un pansement ou un patch, comprenant le produit médical de l'une des revendications 9 à 15 ou 17.
